# EUROPEAN PATENT APPLICATION

(11) **EP 2 151 251 A1**
(43) Date of publication of application: **10.02.2010**
(21) Application number: 09166140.5
(22) Date of filing: 22.07.2009
(51) Int. Cl.: A61L 15/58, A61L 15/24, A61L 15/26, A61F 13/02

(54) **Adhesive patch for protecting and treating viral cutaneous lesions**

(30) Priority: 08.08.2008 IT MI20081512
(71) Applicant: BIOFARMITALIA S.p.A., 20144 Milano (IT)
(72) Inventor: Pinna, Marco, 21051, ARCISATE (VA) (IT)
(74) Representative: Frignoli, Luigi

(57) **Abstract**

An adhesive patch for protecting and treating viral cutaneous lesions, in particular herpes and verrucas, the patch being permeable to gases and vapours and preventing passage of micro-organisms or bacteria therethrough.

## Description

The present invention relates to an adhesive patch, applicable to the skin, for protecting viral cutaneous lesions against external infections, in particular herpes and warts, by forming an effective barrier against external bacteria while enabling skin transpirability at the affected part.

It is known to use patches to treat viral cutaneous lesions ( WALBROEHL G. Treating Periungual Warts with Adhesive Tape. American Family Physician. January 1998,Vol.57,No 2, first page of "Letters to the Editor", SUMMERS. J.B. Is Duct Tape Occlusion Therapy an Effective Treatment of Warts? American Family Physician, November 2003, Vol.68,No 10, pages 2-3 of "Letters to the Editor" and WO 2006/130461,A1 ), but in no case has the objective of these patches been achieved, i.e. providing an absolute barrier to bacterial contamination while at the same time creating conditions suitable for rapid healing of viral lesions.

It has now been found possible to form an extraordinary viral lesion protection associated with a constant limited passage of water vapour, this enabling healing under ideal conditions such as to considerably accelerate healing.

The aforesaid objectives are achieved by an adhesive patch comprising a flexible support, at least a portion of the surface of which is covered with at least one layer of material adhesive towards the skin at a viral lesion, **characterised in that** said flexible support consists of a sheet or film of polymeric material having a thickness between 5 and 300 µm and a permeability to gases and vapours between 150 and 300 g/m², said layer of adhesive material having a permeability to water vapour exceeding 300 g/m², said permeability being measured over a time of 24 hours (this hence being the quantity of water vapour permeated within a time of 24 hours).

Preferably, said sheet or film of polymeric material has a thickness between 5 and 50 µm and is made of a material chosen from the group comprising polyester, PVC, polypropylene, polyurethane, polystyrene, polyethylene and nylon. More preferably, it has a thickness between 10 and 25 µm. According to a preferred aspect, said adhesive material is chosen from the group comprising water-based or solvent-based acrylic adhesives, polyurethane adhesives, resins of natural or synthetic origin, polyacrylates, natural polymers, gums, polyvinyl alcohols, cellulose, carrageenans, alginates. Preferably this adhesive material, after removal of any solvent present therein, forms a layer corresponding to a weight between 10 and 50 g/m².

The polymeric sheet or film forming the strong, flexible support for the patch of the present invention has a permeability such that under static conditions, i.e. the normal use conditions, it is not traversed by micro-organisms, in particular Gram positive pathogens (Staphylococcus aureus and Streptococcus pyogenes), Gram positive and Gram negative commensal bacteria and opportunistic pathogens (Staphylococcus xylosus, Enterococcus faecalis, Escherichia coli, Klebsiella pneumonia, Proteus mirabilis), saprophyte Gram negative bacterial strains and opportunistic pathogens (Pseudomonas aeruginosa) and yeast strains (Candida albicans), not even after a 24 hour prolonged contact.

The nature and characteristics of an example of a highly transpiring adhesive material suitable for use in the patch of the present invention are described in US 6,262,329 (Brunsveld et al.).

Adhesive patches with the aforesaid characteristics have surprisingly demonstrated an effectiveness greater than any other treatment for viral cutaneous lesions by similar known systems, i.e. by plasters with or without the use of drugs.

This circumstance is made possible by the microenvironment created by the rapid passage of vapour and gases through the adhesive material layer (the passage then encountering a slight resistance in passing through the pores of the flexible support), to consequently form a vapour circulation which maintains the injured part under optimal aeriform humidity and flow conditions.

The patch of the invention is such as to maintain these characteristics for an adequate time and can be applied to the surface of a user's viral lesion to accelerate healing without damaging the skin; the patch may or may not contain pharmaceutical substances suitable for such lesions.

According to one aspect of the invention, dispersed within the adhesive material there is at least one natural or synthetic substance having pharmaceutical, aromatic, hydrating, lenitive, softening properties or essential oil, such as chlorhexidine (possessing antibacterial properties), colloidal silver, plant extracts, hyaluronic acid; because of the effect of the adhesive material within which they are dispersed, these substances remain in contact with the cutaneous lesion and facilitate healing. In particular, an antiviral substance can be present, such as acyclovir.

To better understand the structure and use of the patch according to the present invention, a preferred embodiment thereof is described hereinafter by way of nonlimiting example with reference to the accompanying drawings, of which:
Figure 1 is a plan view of a built-up patch prior to use;
Figure 2 is an enlarged-scale cross-section through the patch taken on the line 2-2 of Figure 1; and
Figure 3 is a cross-section through the basic patch, i.e. as to be applied to the skin shown as it is presented at the moment of its use on a cutaneous lesion.

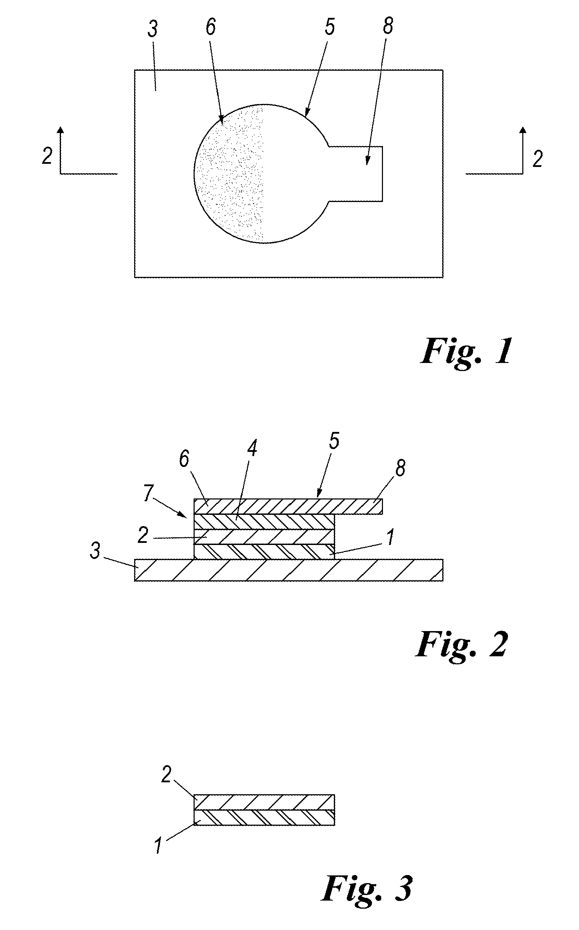

With particular reference to Figure 3, the basic patch comprises the flexible support 2 and the layer of skin-adhesive material 1.

In addition to the structure of the patch as applied to a cutaneous lesion, the invention also relates to the built-up form in which the patch is produced and sold to the user: in this built-up form the free surface of said adhesive material layer 1 rests on a removable sheet 3 of siliconized or polyethylenated paper or siliconized polyester which sheet, for example, can have a thickness of about 100 µm; on that surface of said polymeric material opposite the surface on which said adhesive material is present, there rests a surface of a supplementary film 4 of a polymer preferably chosen from the group comprising polyethylene and polypropylene and preferably having a thickness between 15 and 50 µm, on the other surface of said supplementary film, there resting a shaped sheet 5 glued onto at least one portion of said supplementary film and presenting an appendix 8 without glue which extends outside said supplementary film.

The permeability to water vapour is measurable in accordance with UNI 4848/26.

The present invention is illustrated by the following examples.

### EXAMPLE1

### Preparation of a patch for treating Herpes simplex.

15 kg of solvent based acrylic adhesive (for example Duro-tak 2353 of National Starch & Chemical) are fed cold into a container. Using a patch spreading machine and with the aid of a compressed air pump the acrylic adhesive mixture is transferred onto a rotating cylinder doctor blade, having adjusted the doctor blade thickness to about 60 µm.

The doctor blade spreads the adhesive 1 (to the required thickness) onto the siliconized surface of a continuous tape of plastic material 3, for example 100 µm thick polyester.

The adhesive 1 spread onto the siliconized surface of the polyester 3, is passed at a speed of eight metres per minute through four successive oven stations, the first oven station having its temperature controlled at 40ºC, the second at 50ºC, the third at 70ºC and the fourth at 100ºC.

At the oven exit the adhesive layer 1 is completely free of solvents, which have evaporated in the oven stations; the thickness of the layer of adhesive mass corresponds to a weight of about 20 g/m². This adhesive layer allows a transpirability of 480 g/m² per 24 hours.

At the oven exit, the polyester tape 3 with the adhesive layer 1 applied thereon is coupled to a polyurethane tape 2 which has been previously coupled to a polyethylene tape 4 (15 µm polyurethane extruded on 40 µm polyethylene) of which the polyurethane presents a permeability of about 215 g/m² per 24 hours. The result is that the free surface of the adhesive layer 1, protected on its other surface by the siliconized polyester sheet 3, bonds to the polyurethane tape 2, which is itself coupled to the polyethylene tape 4.

A continuous reel is hence obtained (comprising the layers 1, 2, 3, 4) which is cut into reels having a width of 32 mm.

At this point, using a rotary punching machine, the composite tape 1, 2, 3, 4 is punched to the shape of a circle of 15 mm diameter, then, onto the upper part of the patch, and hence in contact with the polyethylene 4, a shaped sheet of semi-adhesive paper 5 is applied, having an adhesive part 6 which is made to adhere to about one half of the circular surface 7 of the patch, while a non-adhesive appendix 8 of the paper sheet, projects beyond the free edge of the patch (Figures 1 and 2), to act as a gripping element to separate (at the moment of use of the patch, Figure 3) the actual patch 1, 2 from the polyester layer 4. The product (1-5) thus obtained is then automatically inserted into an envelope. To use the patch, its containing envelope is opened and the patch extracted (built up as shown in Figures 1 and 2) together with its polyester protection layer 3, the patch then being separated from this protection layer 3. The adhesive part 1 of the patch is applied to and pressed on the herpes lesion, then the appendix 8 of the semi-adhesive paper sheet 5 is gripped with two fingers and pulled so that, since it is attached (glued) to the surface of the polyethylene 4, it also pulls with it the polyethylene 4, to free the polyurethane layer 2 (Figure 3) which remains attached to the adhesive layer 1 and, via this latter, to the skin.

The patch is replaced every 12 hours until the Herpes has completely healed.

### EXAMPLE 2

### Preparation of a patch for treating warts.

15 kg of solvent based acrylic adhesive (for example Duro-tak adhesive 87-2054 of National Starch & Chemical) are fed cold into a container.

Using a patch mixing/spreading machine, and with the aid of a compressed air pump, the acrylic adhesive mixture is obtained and transferred onto a rotating cylinder doctor blade, having adjusted the doctor blade thickness to about 100 µm. The doctor blade spreads the adhesive 1 (to the required thickness) onto a continuous tape of plastic material 3, for example 100 µm siliconized polyester.

The adhesive 1 spread onto the siliconized polyester 3 is passed at a speed of 8 metres per minute through four successive oven stations, the first oven station having its temperature controlled at 40ºC, the second at 60ºC, the third at 90ºC and the fourth at 120ºC.

At the oven exit the adhesive layer is completely free of solvents, which have evaporated in the oven stations; the thickness of the adhesive mass 1 corresponds to a weight of about 45 g/m², allowing a transpirability of 340 g/m² per 24 hours.

At the oven exit, the polyester tape 3, with the adhesive layer 1 spread thereon, is coupled to a composite polyurethane tape 2 which has been previously extruded and coupled to a polyethylene tape 4 (18 µm polyurethane extruded on a 40 µm polyethylene) of which the polyurethane presents a permeability of about 180 g/m² per 24 hours. The result is that the free surface of the adhesive layer 1, protected on its other surface by the siliconized polyester sheet 3, bonds to the polyurethane tape 2 which is itself coupled to the polyethylene tape 4.

A continuous reel is hence obtained (comprising the layers 1, 2, 3, 4) which is cut into reels having a width of 42 mm.

At this point, using a rotary punching machine, the composite tape 1, 2, 3, 4 is punched to give single elements in the shape of a circle of 20 mm diameter, then applying onto the upper part of the patch, and hence in contact with the polyethylene 4, a sheet of semi-adhesive paper 5 having an adhesive part 6 (in contact with the polyethylene 4), which is adhesive and is made to adhere to about one half of the circular surface 7 of the patch, while a non-adhesive appendix 8 of the paper sheet projects from the circular portion of the patch 1, 2, 4 (Figures 1 and 2) to act as an element to be gripped with two fingers to separate the patch 1, 2 from the polyester layer 4, when the patch (Figure 3) is to be used. The final built-up patch (Figures 1 and 2) is automatically inserted into an envelope. To use the patch, the envelope is opened, the patch of Figures 1 and 2, still possessing its polyester protection layer 3 is extracted, and this protection layer 3 is removed. The adhesive part 1 of the patch is applied to and pressed on the wart lesion, then after its application the appendix 8 of the semi-adhesive paper sheet 5 is pulled with two fingers, which appendix, being attached (glued) to the surface of the polyethylene 4, it also removes the polyethylene layer 4, to free the polyurethane layer 2 of thickness 18 µm and enable the finished patch 1, 2 (Figure 3) to be used.

The patch 1,2 is replaced every 12 hours until the wart has completely healed.

### EXAMPLE 3

### Preparation of a patch for treating labial herpes with pharmaceutical active substance.

10 kg of solvent based acrylic adhesive (for example Duro-tak adhesive - 2353 of National Starch & Chemical) are fed cold into a container.

This adhesive is fed into a screw mixer. The screw is rotated at a speed of 60 revolutions per minute and 182.5 g of Acyclovir (antiviral pharmaceutical substance amino-1,9-dihydro-[(2-hydroxyethoxy) methyl-6H-pyrin-one) are introduced and rotation is continued at ambient temperature for 15 minutes. Rotation is halted and the mixture left at rest for 120 minutes to deaerate.

Using a patch mixer/spreading machine and with the aid of a compressed air pump, the acrylic adhesive mixture obtained is transferred onto a rotating cylinder doctor blade, having adjusted the doctor blade thickness to about 60 µm.

The doctor blade spreads the adhesive 1 (to the required thickness) onto a continuous web of plastic material 3, for example 100 µm siliconized polyester.

The adhesive 1 spread onto the polyester is passed at a speed of eight metres per minute through four successive oven stations, the first oven station having its temperature controlled at 40ºC, the second at 50ºC, the third at 70ºC and the fourth at 100ºC.

At the oven exit the adhesive layer is completely free of solvents, which have evaporated in the oven stations; the thickness of the adhesive mass corresponds to a weight of about 20 g/m², allowing a transpirability of 480 g/m² per 24 hours.

At the oven exit, the polyester web 3 with, the adhesive layer 1 spread thereon, is coupled to a composite polyurethane tape 2 which has been previously extruded and coupled to a polyethylene tape 4 (15 µm polyurethane extruded on a 40 µm polyethylene), of which the polyurethane presents a permeability of about 215 g/m² per 24 hours. The result is that the adhesive layer 1, protected on one side by the siliconized polyester sheet 3, bonds to the polyurethane tape 2 (which is itself coupled to the polyethylene tape 4).

The reel obtained (comprising the layers 1, 2, 3, 4) is then cut into reels having a width of 32 mm.

At this point, using a rotary punching machine, the coupled assembly is punched to give composite elements in the shape of a circle of 15 mm diameter, then, onto the upper part of the patch, and hence in contact with the polyethylene 4, a sheet of semi-adhesive paper 5 is applied, having an adhesive part 6 (in contact with the polyethylene 4), which is glued to about one half of the patch circle, while the non-adhesive part projects from the patch edge to form an appendix 8 (Figures 1 and 2) to be gripped with the fingers to separate the patch. The protected patch cut in this manner is then automatically inserted into an envelope. To use the patch 1, 2 (Figure 3) the envelope is opened, the patch with its polyethylene protection layer 3 is extracted, and the patch separated from this protection layer 3. The adhesive part 1 of the patch is applied to and pressed on the Herpes lesion, then after its application the appendix 8 of the semi-adhesive paper sheet 5 is pulled with two fingers, which sheet, being attached to the polyethylene layer 4, it also removes the polyethylene layer, to free the polyurethane layer 2 of thickness 15 µm and enable the finished patch 1, 2 (Figure 3) to be used.

The patch 1,2 is replaced every 12 hours until complete healing has taken place.

### EXAMPLE 4

### Preparation of a patch with vegetable extract for treating warts.

15 kg of solvent based acrylic adhesive (for example Duro-tak adhesive - 2054 of National Starch & Chemical) are fed cold into a container. This adhesive is fed into a screw mixer. The screw is rotated at a speed of 60 revolutions per minute and 300 g of fluid grapefruit seed extract are introduced and rotation is continued at ambient temperature for 15 minutes. Rotation is halted and the mixture left at rest for 120 minutes to deaerate.

Using a patch spreading machine and with the aid of a compressed air pump the acrylic adhesive mixture obtained in this manner is transferred onto a rotating cylinder doctor blade, having adjusted the doctor blade thickness to about 60 µm.

The doctor spreads the adhesive 1 (to the required thickness) onto a continuous tape of plastic material, for example 100 µm siliconized polyester or siliconized paper.

The adhesive spread onto the polyester is passed at a speed of eight metres per minute through four successive oven stations, the first oven station having its temperature controlled at 40ºC, the second at 50ºC, the third at 70ºC and the fourth at 100ºC.

At the oven exit, the adhesive layer is completely free of solvents, which have evaporated in the oven stations; the thickness of the adhesive mass 1 corresponds to a weight of about 20 g/m², allowing a transpirability of 340 g/m² per 24 hours.

At the oven exit, the polyester tape 3, with the adhesive layer 1 applied thereto, is coupled to a polyethylene tape of 15 µm, presenting a permeability of about 250 g/m² per 24 hours. The result is that the adhesive layer 1, which is protected on one of its sides by the siliconized polyester sheet 3, bonds to the polyethylene tape.

The reel obtained is then cut into reels having a width of 40 mm.

At this point, using a rotary punching machine, the coupled assembly is punched to give composite elements in the shape of a circle of 20 mm diameter. The patch cut in this manner is then automatically inserted into an envelope. For application, the envelope is opened, the patch with its polyester protection layer is extracted, and the patch separated from this protection layer. The adhesive part of the patch is applied to the wart lesion.

The patch is replaced every 12 hours until complete healing has taken place.

## Claims

1. An adhesive patch to be used for protecting and treating viral cutaneous lesions, comprising a flexible support (2), at least a portion of the surface of which is covered with at least one layer (1) of material adhesive to the skin at a viral lesion, **characterised in that** said flexible support consists of a sheet or film of polymeric material having a thickness between 5 and 300 µm and a permeability to water vapour between 150 and 300 g/m², said layer of adhesive material having a permeability to water vapour exceeding 300 g/m², said permeabilities being measured over a time of 24 hours.

2. A patch as claimed in claim 1, **characterised in that** said sheet or film of polymeric material has a thickness between 5 and 50 µm and is made of a material chosen from the group comprising polyester, PVC, polypropylene, polyurethane, polystyrene, polyethylene and nylon.

3. A patch as claimed in claim 2, **characterised in that** said sheet or film of polymeric material has a thickness between 10 and 25 µm.

4. A patch as claimed in claim 1 or 2 or 3, **characterised in that** said adhesive material is chosen from the group comprising water-based or solvent-based acrylic adhesives, polyurethane adhesives, resins of natural or synthetic origin, polyacrylates, natural polymers, gums, polyvinyl alcohols, cellulose, carrageenans, alginates, said adhesive material, after removal of any solvent present therein, forming a layer corresponding to a weight between 10 and 50 g/m².

5. A patch as claimed in claim 1, 2, 3 or 4, **characterised in that** at least one substance having pharmaceutical, hydrating, lenitive or aromatic properties or in the form of essential oils, colloidal silver, hyaluronic acid or plant extract, is dispersed within said adhesive material.

6. A patch as claimed in claims from 1 to 5, **characterised by** being built up into a form such that the free surface of said adhesive material layer 1 rests on a removable sheet (3) of siliconized or polyethylenated paper or siliconized polyester, on that surface of said polymeric material opposite to the surface on which said adhesive material is present, there resting a surface of a supplementary film (4) of polymeric material, on the other surface of said supplementary film there resting a shaped sheet (5) glued onto at least one portion of said supplementary film and presenting an appendix (8) without glue which extends outside said supplementary film.
